# EUROPEAN PATENT SPECIFICATION

(11) **EP 3 091 831 B1**
(45) Date of publication and mention of the grant of the patent: **23.12.2020**
(21) Application number: 15700539.8
(22) Date of filing: 09.01.2015
(51) Int. Cl.: A01H 1/04, C12Q 1/68

(54) **EMBRYO SAMPLING METHOD**
PROBENAHMEVERFAHREN FÜR EMBRYOS
MÉTHODE DE PRÉLÈVEMENT D'EMBRYONS

(30) Priority: 09.01.2014 US 201461925277 P
(43) Date of publication of application: 16.11.2016
(73) Proprietor: Syngenta Participations AG, 4058 Basel (CH)
(72) Inventor: BULLOCK, William Paul, Slater, Iowa 50244 (US); HANNAPPEL, Ulrich Stephan, Slater, Iowa 50244 (US); HUNTER, Katie Marie, Slater, Iowa 50244 (US); HEJLIK, Christopher Cody, Slater, Iowa 50244 (US)
(74) Representative: SYNGENTA IP
(86) International application number: PCT/EP2015/050299
(87) International publication number: WO 2015/104358

(56) References cited:
- WO-A1-2011/019863
- WO-A1-2014/195199
- UJJAL KUMAR NATH ET AL: "Early, non-destructive selection of microspore-derived embryo genotypes in oilseed rape (Brassica napus L.) by molecular markers and oil quality analysis", MOLECULAR BREEDING, KLUWER ACADEMIC PUBLISHERS, DO, vol. 19, no. 3, 13 January 2007 (2007-01-13), pages 285-289, XP019482977, ISSN: 1572-9788, DOI: 10.1007/S11032-006-9053-Y
- PEGGY HORN ET AL: "Non-destructive RAPD genetic diagnostics of microspore-derivedBrassica embryos", PLANT MOLECULAR BIOLOGY REPORTER, SPRINGER SCIENCE+BUSINESS MEDIA B.V, NL, vol. 10, 1 January 1992 (1992-01-01), pages 285-293, XP009182767, ISSN: 0735-9640
- RÖBER F K ET AL: "In vivo haploid induction in maize - Performance of new inducers and significance of doubled haploid lines in hybrid breeding", MAYDICA, ISTITUTO SPERIMENTALE PER LA CEREALICOLTURA, BERGAMO, IT, vol. 50, 1 January 2005 (2005-01-01), pages 275-283, XP002508662, ISSN: 0025-6153
- ROGERS S O ET AL: "EXTRACTION OF DNA FROM MILLIGRAM AMOUNTS OF FRESH HERBARIUM AND MUMMIFIED PLANT TISSUES", PLANT MOLECULAR BIOLOGY, SPRINGER, DORDRECHT, NL, vol. 5, no. 2, 1 January 1985 (1985-01-01) , pages 69-76, XP009158611, ISSN: 0167-4412

## Description

Maize plant breeding has increasingly become more dependent upon a) doubled haploid (DH) processes to rapidly create true breeding inbred parental lines, b) the consolidation of desired quantitative trait loci into individual corn lines, and c) the introgression of transgenes into desired commercial inbreds. With respect to these goals, one of the challenges in commercial plant breeding is the rapid acceleration of the above described breeding process. In particular, the inability to make breeding or genetic selections based on plant genotype or biochemical constitution/makeup soon after seed development imposes time, resource and consumable costs and constraints. For example, the postponement of breeding advancement or other selection decisions creates significant management challenges, because large populations of individual seeds and plants must be carried forward due to the inability to make data driven selection decisions earlier in the breeding pipeline.

This present application illustrates methods which 1) provide for very early identification of genetically or compositionally selected individuals and 2) sustains the viability/germination capacity of these individuals. More specifically, scutellar and/or embryo axis tissue or cellular contents derived from immature corn (about 5 to 24 days post pollination, the range of which is partially dependent upon environmental growing conditions) embryos are assayed to determine their genotype or biochemical composition. Post tissue sampling or cellular content removal, the resulting embryos are quite capable of germinating and developing into normal corn plants.

The literature alludes to DNA extraction from embryos, but do not indicate how this might effectively be done. For example, Jordano & Godoy, in "Lab protocols for DNA extraction and genotyping of SSR microsatellites for Prunus mahaleb (Rosaceae) seeds and Quercus acorns" (2005) describe the extraction of DNA from entire embryos and so no viability is retained. Sparks et al., Plant Physiology December vol. 133 no. 4 1809-1819 (2003) also describe DNA extraction from embryos. Again, viability is not retained. Patent application WO2011/019863 A1 discusses automated embryo extraction from corn seeds. However, it does not claim or describe "retained viability" of the embryos after tissue removal. The patent does not describe any specific method as to how to isolate cells from embryos. Patent US 8312672 describes the problem, but proposes to analyze endosperm tissue (via seed chipping) to fingerprint the embryo.

The tissue sampling and cellular content sampling processes described below provide for 1) early acquisition of genetic and biochemical data from which to make data driven breeding decisions, 2) processes for tissue and cellular content sampling, 3) minimization of requisite DNA purification steps, 4) reduced cost per DNA extraction, 5) allows for the direct use of the resulting DNA for PCR analysis, 6) a viability retaining tissue sampling and cellular content sampling method targeting very immature plant tissues and immature corn embryos, and 6) methods which are readily automated.

The methodology that the inventors of the present application have developed illustrates how DNA extraction might effectively be done. The methods can be categorized by extraction methodology: 1) mild chemical disruption of cells/organized tissues such that DNA and other cellular content is eluted from some embryo tissue into the surrounding solution and 2) mechanical means which remove tissue and/or cellular contents from the scutellum or embryo axis regions of immature corn embryos. Uniquely, each of these extraction methods provides for viability-retaining germination capacity within the sampled immature embryos.

In general terms, the methodology includes a series of steps: 1) Immature embryo extraction, 2) Tissue/DNA/cellular content extraction by either a) soaking of an embryo in solution that provides for DNA and cellular content leakage from cells/organized tissues, or b) electroporation of immature embryos so as to cause leakage of cellular contents or c) sampling of tissue from immature scutellum by plugging, or penetrations or abrasion or scraping, or slicing 3) the option for automation of the aforementioned processes, and 4) high rates of embryo germination. As a series of steps, the inventors provide various methods for a viability-retaining tissue/DNA and cellular content extraction processes that provides the opportunity to make genetic data driven selection decisions in one of the earliest possible manners.

### SCOPE OF THE INVENTION

There is provided a non-destructive, viability retaining method of sampling an immature plant embryo, comprising the step of removing a tissue sample comprising at least a portion of the scutellum, wherein the obtained cellular content via tissue sampling is subjected to genetic, chemical or biochemical analysis, and wherein the extracted embryos are put into stasis storage to achieve a quiescent condition until the outcome of the genetic, chemical or biochemical analysis is known. In one embodiment, the embryo is a haploid embryo. In one embodiment, the embryo is a diploid embryo.

There is also disclosed a non-destructive, viability retaining method of sampling a plant embryo, comprising the step of releasing and collecting cells or directly releasing the cellular contents from the scutellum and/or embryo axis region of the immature embryo. In one embodiment, the immature embryo is a corn immature embryo. In one embodiment, the embryos are extracted from ears of corn at 8 to 24 days post pollination. In one embodiment, the embryos are approximately 1 to 6 mm. In a preferred embodiment, the embryos are 2 to 6 mm.

In one embodiment, the method according to the invention further comprises the step of performing analysis on the tissue sample or cellular contents from the embryo.

In one embodiment, the method according to the invention further comprises the step of selecting an embryo based on the result of the analysis.

In one embodiment, the method according to the invention is at least semi-automated. In one embodiment, the method according to the invention is automated.

In one embodiment of the method according to the invention, the embryo is at the non-mature stage of development.

In one embodiment of the method according to the invention, the sample removed comprises tissue from the coleoptilar end of the embryo.

In one embodiment of the method according to the invention, the sample removed comprises tissue from the non-coleoptilar end of the embryo.

In one embodiment of the method according to the invention, the sample removed comprises tissue from the equator or middle region of the embryo.

In one embodiment of the method according to the invention, the sample is removed using a penetrant. In one embodiment, the penetrant is a toothpick. In one embodiment, the penetrant is a pipette tip. In one embodiment, the penetrant is a solid needle. In one embodiment, the penetrant is a hollow needle. In one embodiment, the penetrant is a capillary tube.

In one embodiment, the sample is removed using a charged penetrant which attracts or retains tissues and/or cellular content e.g. DNA. In one embodiment, the penetrant has a roughened surface. In one embodiment, the penetrant has a smooth surface.

In one embodiment of the method according to the invention, cells are removed using sandpaper, or similar abrasive material in order to release cells and cellular contents and optimize their retention on the abrasive material.

In one embodiment cellular content is directly sampled onto material which selectively captures components of the cells.

In one aspect of the method according to the embodiment, the cellular content is removed by cell disruption using a liquid solution to bathe the embryo for a sustained period of time. In one embodiment, the liquid is NaOH. In one aspect the liquid is KOH. In one aspect the liquid is TrisHCl. In one aspect the liquid is SDS. In one aspect the liquid is Tween 20.

In one aspect the solution contains the buffering tris (hydroxymethyl) aminomethane (Tris). In one aspect the solution contains 4-(2- hydroxyethyl) -1-piperazineethanesulfonic acid (HEPES). In one aspect the solution contains the buffering component 3- (N- morpholino) propanesulfonic acid (MOPS). In one aspect the solution contains the buffering component sodium dihydrogen phosphate (NaH2 P04). In one aspect the solution contains the buffering component disodium hydrogen phosphate (Na2HP04).

In one aspect the solution contains the detergent SDS. In one aspect the solution contains the detergent Triton X-100. In one aspect the solution contains the detergent Triton X-114. In one aspect the solution contains the detergent NP-40. In one aspect the solution contains the detergent Brij-35. In one aspect the solution contains the detergent Brij-58. In one aspect the solution contains the detergent Tween 20. In one aspect the solution contains the detergent Octyl glucoside. In one aspect the solution contains the detergent CHAPS. In one aspect the solution contains the detergent CHAPSO.

In one aspect, the liquid comprises a combination of a buffer and a detergent selected from the lists above.

In one aspect the cell disruption can be performed with a solution containing the enzyme cellulase. In one aspect the cell disruption can be performed with a solution containing the enzyme pectinase. In one aspect the cell disruption can be performed with a solution containing the enzyme beta-glucanase. In one aspect the cell disruption is performed with a solution containing the enzyme mannanase. In one aspect the cell disruption is performed with a solution containing the enzyme xyloglucanase. In one aspect the cell disruption is performed with a solution containing the enzyme glycosidase. In one aspect the cell disruption is performed with a solution containing the enzyme xylanase. In one aspect the cell disruption is performed with a solution containing a mixture of at least two enzymes from above.

In one aspect the solution contains the additive component EDTA. In one aspect the solution contains the additive component EGTA. In one aspect the solution contains the additive component PVP. In one aspect the solution contains the additive component PEG.

In one aspect the cell disruption is performed at a temperature between 10 and 30 degree Celsius. In one aspect the cell disruption is performed between 5 minutes and 24 hours.

In one aspect of the method according to the disclosure, the cellular content is removed from the immature corn embryo by cell disruption using gentle sonication and optionally, cell wall degrading enzymes.

In one aspect cells are disrupted by an electric field by using an electroporator or any other electronic device which disrupts cell membranes. Cell membranes may be disrupted permanently or a made porous for a short period of time allowing for the release of cellular content.

In one embodiment scutellum tissue is removed mechanically (razor blade, laser or any other tool). The removed tissue is discarded and the mechanically manipulated embryos are placed into a liquid to collect cellular content. In one embodiment, the liquid may degrade cell membranes and walls. In one embodiment, cell wall degradation is not required. In one embodiment the cellular content of embryos or microspore derived embryos is not captured in liquid but on any structure (e.g. filter paper) which selectively binds all or a specific compound of the cellular content.

In one embodiment, the method according to the invention further comprises the collection of cells and cellular contents from the liquid or cellular content binding solid structure.

In one embodiment of the method according to the invention, the sample is removed by slicing with a knife or scalpel or a laser or similar devices.

In one embodiment of the method according to the invention, the removed sample is subjected to genetic, chemical or biochemical analysis.

In one embodiment of the method according to the invention, the embryo is a maize embryo.

In one embodiment of the method according to the invention, the embryo is a wheat embryo.

In one embodiment of the method according to the invention, the embryo is a rice embryo.

The concept could be applied to somatic embryos derived from microspore culture.

In one embodiment, the embryo is a microspore derived embryo from a Brassica species. In one embodiment, the embryo is a microspore derived embryo from a Hordeum species. In one embodiment, the embryo is a microspore derived embryo from a Cucumis species. In one embodiment, the embryo is a microspore derived embryo from a Zea Mays species. In one embodiment, the embryo is a microspore derived embryo from a Triticum species. In one embodiment, the embryo is a microspore derived embryo from a Capsicum species. In one embodiment, the embryo is a microspore derived embryo from an Oryza species. In one embodiment, the embryo is a microspore derived embryo from a Saccharum species.

There is also disclosed a plant embryo, wherein said embryo has been sampled according to the method of the disclosure.

There is also disclosed the use of a plant embryo according to the disclosure in plant production.

There is also disclosed a maize plant produced from an embryo according to the disclosure.

There is also provided a method of data driven plant advancement, comprising the steps of a) inducing haploids; b) extracting and identifying haploid immature embryos from amongst a mixed population of diploid and haploid embryos; c) obtaining a tissue sample preferably from the scutellum from the extracted embryos in a non-destructive manner, said samples comprising at least a portion of the scutellum; and d) optionally, doubling chromosome number; wherein step c) can take place before and/or after step d).

In one embodiment, the method of the invention further comprises one or more of the steps of e) analyzing the genetic, chemical or biochemical characteristics of the sampled embryos; f) advancing selected embryos in a breeding process based on the analyzed characteristics; and g) producing seedlings from the selected embryos. There is also provided a method of data driven plant advancement, comprising the steps of h) obtaining a tissue sample from the scutellum tissue, from the scutellum of the extracted diploid embryos in a non-destructive manner, i) analyzing the genetic, chemical or biochemical characteristics of the sampled embryos; j) advancing selected embryos in a breeding process based on the analyzed characteristics; and k) producing seedlings from the selected embryos.

In one embodiment, the method of the invention further comprises the production of seeds from the seedlings of steps g) and k)

In one embodiment of the method according to the invention, the extracted embryos are put into stasis storage to achieve a quiescent condition until the outcome of the genetic, chemical or biochemical analysis is known.

In one embodiment of the method according to the invention, the embryos are stored in a controlled atmosphere. In one embodiment of the method according to the invention, the embryos are stored at a controlled temperature. In one embodiment of the method according to the invention, the embryos are stored under pre-determined nutritional conditions. In one embodiment of the method according to the invention, the embryos are stored in a controlled atmosphere and temperature and pre-determined nutritional conditions.

In one embodiment, stasis storage controls cell division in a productive fashion to the benefit of chromosome doubling. This equates to better fertility and seed production.

In one embodiment of the method according to the invention, the embryos are stored in the presence of anti-ethylene or ethylene metabolism impacting compounds which reduce the production of, or bind with and remove, or obviate the impact ethylene production. In one embodiment, these are compounds which bind to the same receptor sites to which ethylene also binds. In one embodiment, the compound is silver nitrate. In one embodiment, the compound is silverthiosulfate. In one embodiment, the compound is 1-MCP. In one embodiment, the compound is aminoethoxyvinylglycine. In one embodiment, the compound is 2,5-Norbornadiene. In one embodiment, potassium permanganate is used to bind gaseous ethylene. In one embodiment, the compound is an anti-ethylene or ethylene metabolism impacting compounds known to the skilled person which reduce the production of, or bind with and remove, or obviate the impact ethylene production.

In one embodiment of the method according to the invention, the embryo is manipulated using a robotic arm system; a camera is used to select haploids or non-haploids; and a camera or vision system is used to guide the sampling instrument to the tissue to be sampled.

There is also provided a method of haploid embryo discrimination, comprising the steps of capturing an image of a population of embryos; identifying haploid embryos in said population; moving selected embryos via transporting means.

In one embodiment, the method of the invention further comprises the steps of transferring all embryos or selected embryos to a different location using said transporting means.

In one embodiment, the method of the invention further comprises the steps of placing the attached embryos such that tissue sample(s) can be removed from the embryos; transferring all embryos or selected embryos to a different location. In one embodiment, the location is a storage location, preferably stasis storage.

In one embodiment, chromosome doubling is applied to immature seedlings post stasis storage.

In one embodiment, the method of the invention further comprises the steps of placing the attached embryos such that a tissue sample can be removed from the embryos; transferring all embryos or selected embryos to a controlled atmosphere and temperature and nutritional environment suitable for embryo storage.

In one aspect, the embryo is moved so that it is contacting a solution so that the cells can be disrupted and the cellular content can be collected.

In one embodiment of the invention, the embryos are identified using algorithms based on either color or spectral analysis.

In one embodiment of the invention, the method is at least semi-automated.

In one embodiment of the invention, the tissue sampling steps are automated.

### DEFINITIONS

The technical terms and expressions used within the scope of this application are generally to be given the meaning commonly applied to them in the pertinent art of sampling biological materials if not otherwise indicated herein below.

Immature Haploid Corn Embryo: A haploid embryo (n=10) composed of a central embryo axis and scutellum before its typical full level of development in the kernel and prior to its germination.

Premature Haploid Embryo Germination: the initiation of growth and/or extrusion of the coleorhiza and/or coleoptile axes from the immature haploid embryo post excision of the haploid embryo from the seed.

Immature - Precocious Haploid Seedling: An immature plant-seedling formed from the germination of an excised immature haploid embryo.

Microspore Derived Embryo: an embryo derived from an embryogenic developmental process initiated from a plant microspore.

Scutellum: a modified cotyledon and is a component part of the monocotyledoneus embryo. It is attached to the embryo axis in the scutellar node. It acts as a food storage organ and serves to digest and transport the endosperm nutrients during germination.

Embryo axis: Embryo axis is formed by the primary root, protected by the coleorhiza, and leaf primordia (plumule), protected by the coleoptile.

Coleoptile: The first true leaf and is modified to act as a protective covering for the plumule or first bud of the plant and grows throughout the soil during germination.

Immature Embryo: An embryo which has not yet undergone a desiccation process and has not achieved an associated quiescent state. Immature embryos can be extracted from the seed or caryopsis either by hand or via automation within a matter of days post pollination and which under the appropriate conditions are capable of germination.

Mature Embryo: A corn embryo which has undergone the desiccation process and achieved an associated quiescent state.

Sampling of Immature Embryo Tissue: removal of a portion, piece, or segment of the immature corn zygotic or microspore derived embryo which is representative of the biochemical or genetic constitution of the immature zygotic or microspore derived embryo

Sampling of Cellular Content: removal of cellular content from the immature embryo zygotic or microspore derived embryo which is representative of the biochemical or genetic constitution of the immature corn zygotic or microspore derived embryo

Scutellum coleoptilar end: The area or region of the scutellum which surrounds or is adjacent to the coleoptile.

Scutellum non-coleoptilar end. The area or region of the scutellum which surrounds or is adjacent to the coleorhiza.

Embryonic Axis: The embryo axis is formed by the primary root, protected by the coleorhiza, and the stem tip, protected by the coleoptile.

Tissue Sample: A piece of tissue which is removed from an immature zygotic or microspore-derived embryo. The tissue sample is used to analyze the cellular content which reflects that of the immature zygotic or microspore derived embryo.

Cellular content sampling: Sampling cellular content from an immature zygotic or microspore derived embryo excluding the step of tissue sampling. Cellular content is obtained by disrupting cell membranes of zygotic or microspore derived embryos.

Plugging: Removal of plug tissue from an embryo.

Cellular Contents: Internal constituents of a plant cell which may include DNA or other cytoplasmic components such as organelles, RNA, protein, oils, sugars, starch or other organic compounds.

Penetrant: A physical structure which is capable of penetrating into plant tissue. The penetrant may be solid or hollow. It may contain a smooth or abrasive or charged surface to facilitate the removal of tissue and cellular contents from plant embryos.

Tissue Plug: A tissue sample collected via the penetration of embryo tissue by a hollow penetrant.

Abrasive Surface: A surface which when rubbed or placed in contact with the desired plant surface is capable of abrading the zygotic or microspore derived embryo so as to remove a tissue sample.

Haploid Induction: A plant process whereby the creation of haploid maize zygotic embryos occurs. In maize, various haploid inducing lines (e.g. RWK, RWS, Stock 6, MHI, etc.) can be used as male parents to pollinate other corn germplasm from which maternal haploid embryos are produced. In Wheat, corn pollen is used to stimulate haploid wheat embryo development.

Viability Retaining: Describes the circumstance wherein a plant embryo remains viable and capable of germination post removal of tissue (tissue sampling and/or the disruption of cells).

Non-destructive manner: A method of tissue sampling and/or the disruption of cells which provides for the viability and germination capacity of an immature zygotic or microspore derived embryo.

Storage of Embryos: Storage of immature zygotic or microspore derived zygotic embryos in conditions which minimize respiration and/or metabolic activity in order to maintain the embryos in a viable-quiescent condition.

Stasis Storage: Storage of a plant embryo in a quiescent condition for a sustained period of time (about 1-20 days).

Quiescent Condition: A state of suspended growth or resting condition of the embryo in which metabolic activity is substantially slowed.

Controlled Atmosphere: An atmospheric composition(s) which reduces or minimizes respiration and/or metabolic activity so as to provide for the storage of immature zygotic or microspore derived embryos in a quiescent condition.

Haploid Embryo: This references the genetic ploidy of a zygotic or microspore derived embryo and more specifically said embryos contains half the normal number of chromosomes.

Doubled Haploid Seedling: A zygotic or microspore derived seedling to which a chromosome doubling process has been applied. The seedling may consist entirely or partially of cells which contain a diploid number of chromosomes.

Automated: A process or process step or series of process steps which is or are done entirely by machine.

Semi-automated: A process or process step or series of process steps which is or are done in part by machine and in part by a human being.

Robotic Arm System: A computer controlled electro-mechanical device with multiple degrees of freedom which provides for a simulation of the capabilities of a human arm and hand such that embryos can be moved from one location to another. Furthermore, the robotic arm system provides a means for the taking of a tissue sample from previously describe embryos and transport of said tissue sample(s) and their placement in second location.

Vision system: A camera system capable of distinguishing human visible or multispectral characteristic of zygotic or microspore derived plant embryos. The aforementioned camera system provides information to a computer controlled device such as a robotic arm to facilitate automated process steps.

Transporting Means: This refers to the use of a robotic arm or other mechanical device which is used to move zygotic or microspore derived embryos from one location to another.

Algorithms: A process or set of rules to be followed in calculations or other problem-solving operations, especially by a computer.

Data Driven Plant Advancement/Selection Decision: Use of phenotypic or genetic or biochemical data derived from an individual or groups of individuals or populations in order to select individual(s) or groups of individuals or populations for continued breeding or genetic enhancement efforts or further examination.

As used in this specification and the appended claims, the singular forms "a", "an", and "the" include plural referents unless the context clearly dictates otherwise. Thus, for example, reference to "a plant" includes one or more plants.

As used herein, the term "about" when referring to a value / amount of mass, weight, time, volume, concentration or percentage is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed method.

As used herein, the term "breeding", and grammatical variants thereof, refer to any process that generates a progeny individual. Breeding can be sexual or asexual, or any combination thereof. Exemplary nonlimiting types of breeding include crossings, self-pollination, doubled haploid derivative generation, polyploidization and combinations thereof.

A "plant" is any plant at any stage of development.

A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant.

As used herein, the term "progeny" refers to the descendant(s) of a particular cross or individual. Typically, progeny result from breeding of two individuals, although some species (particularly some plants) can be self-pollinated (i.e. the same plant acts as the donor of both male and female gametes). The descendant(s) can be, for example, of the F1, the F2, or any subsequent generation.

### EXAMPLES

The following examples are presented in order to more fully illustrate some embodiments of the invention.

Below are provided examples of 1) chemical elution of DNA from immature corn embryos, 2) mechanical methods of tissue extraction from scutellar and embryo axis tissue of immature corn embryos, 3) PCR-based genotyping results using the extracted DNA from individuals, and 4) germination capacity of embryos having already received the aforementioned chemical or mechanical treatments.

### Example 1. Ear Sterilization and Embryo Rescue

Ears of corn of varying ages (about 8 to 24 days post pollination) are sterilized in a 50% bleach solution and then rinsed with sterile water. Kernel caps are removed with a scalpel and immature zygotic embryos of various sizes [about 4-6 mm in length] are extracted by hand and placed either into a petri dish containing a filter paper saturated with Murashige and Skoog (M.S., 1962) based liquid growth medium or directly onto the surface of M.S. based solidified growth medium. The embryos are then cultured under grow lights (16 hr. day length, 400 umol, 31C/87F day 22C/72F night).

Diploid embryos may be used directly for tissue extraction or cell disruption. Alternatively and when a scutellar pigmentation marker such as R1scm2 or R1scm3 or R1scm122, or R1scm124 are employed, a short period of time is provided to allow for the discrimination of haploid from diploid embryos. Diploid or haploid embryos are then provided for tissue sampling or cell disruption as described below.

### Example 2. Evaluation of the Germination Capacity of Haploid and Diploid Immature Corn Embryos.

Control or tissue sampled haploid and diploid immature corn embryos are placed onto the surface of solidified M.S. based growth medium and cultured for under the following growing conditions: 16 hr. day length, 400 umol PAR light, 31C/87F day 22C/72F night). Embryo germination is scored after a period of 3 to 5 days.

### Example 3. Soaking of Haploid Embryos in NaOH to Facilitate DNA Extraction.

Experiment 1. For the removal of adherent endosperm tissue, all embryos were pooled and transferred into a 50 ml tube containing 40 ml MS buffer and 3% sucrose. To enhance the washing efficiency, the tube was placed on a horizontal shaker and vigorously shaken for 40 minutes. After 40 minutes the washing solution was poured off and the embryos were briefly rinsed in H₂O.

For DNA extraction, each embryo was placed into a well of a 96 well block, to each embryo 100 µl of a 5 mM NaOH solution was added. The NaOH conc. could vary between approximately 2.5 mM and approximately 50 mM. The soaking time could vary between approximately 20 minutes and approximately 3 hours. Any other chemical which disrupts cells but does not harm the viability of the embryos could be used (e.g. Potassium Hydroxide (KOH)) or any chemical substances which do not harm the viability of the embryos could be added to a cell disrupting solution.

The 96 well block was placed on a horizontal shaker, the blocks were slowly agitated for 1 hour. The alkali solution disrupts scutellar or embryo axis cells, the DNA and other cellular content of the disrupted cells is released into the solution. After 1 hour, 80 µl of the soaking solution from each well were transferred into the wells of a second block and 1M TrisHCl (or any other pH adjusting chemical which does not harm downstream application of the DNA) was added to lower the pH to around 8. Other downstream applications may require different pH adjustments.The embryos were washed several times in MS buffer containing 3% sucrose.

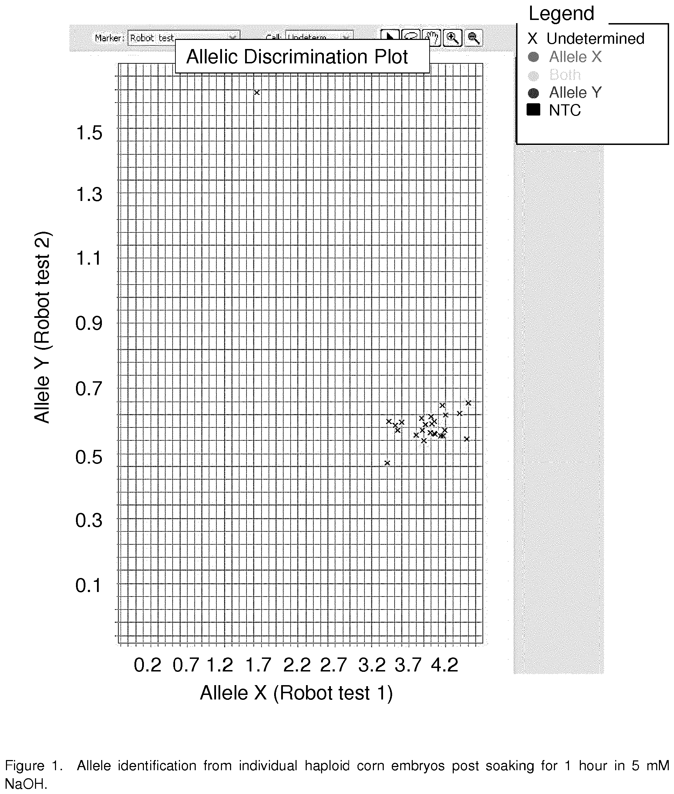

For molecular marker analysis, the DNA from 24 embryos which was released into the soaking solution was used directly as a template for Taqman PCRs. At this point, any DNA cleaning processes could be applied. Modification of the DNA (e.g. additional cleaning steps via alcohol precipitation) could be applied. Direct PCR results are shown in Fig.1

### Experiment 2.

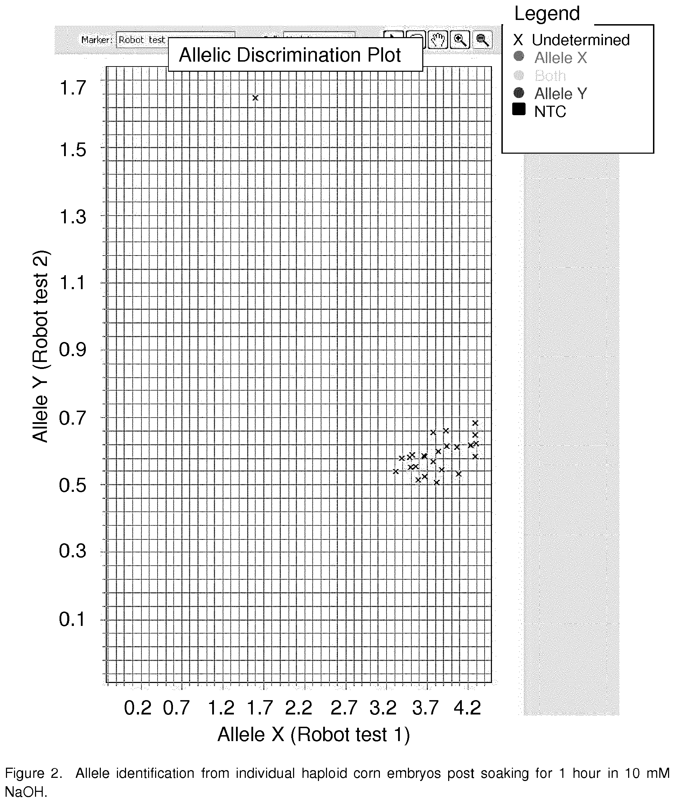

This experiment was performed as described under experiment 1, but DNA was extracted by soaking embryos in 10 mM NaOH. The result for the molecular marker analysis is shown in Fig. 2.

**Table 1. Embryo germination rates observed from embryos soaked in NaOH (5, 10 mM) for 1 hour.**

| **Embryo Ploidy** | **DNA Extraction Method** | **Initial Number of Embryos** | **Number Germinated Embryos** | **Percent Embryo Germination** |
|---|---|---|---|---|
| Haploid | Control | 541 | 534 | 99% |
| Haploid | Embryos soaked in 10mM NaOH for 1 hr. | 29 | 29 | 100% |
| Haploid | Embryos soaked in 5mM NaOH for 1 hr. | 31 | 31 | 100% |

### Example 4. Methods of Endosperm Tissue Removal from Haploid Immature Corn Embryos.

### Endosperm Wash Method 1.

Haploid embryos representing two different F1 hybrids were independently pooled and washed for 5 minutes in 10 mM NaOH and for an additional 15 minutes in MS buffer plus 3% sucrose. Any liquid chemical (e.g. tween 20) can be used to cleanse the embryos as long as the embryo viability is not harmed by the cleansing process. Each washing step was performed in a 50 ml tube. The tube was shaken vigorously on a horizontal shaker. After both washing steps the embryos were rinsed in H₂O.

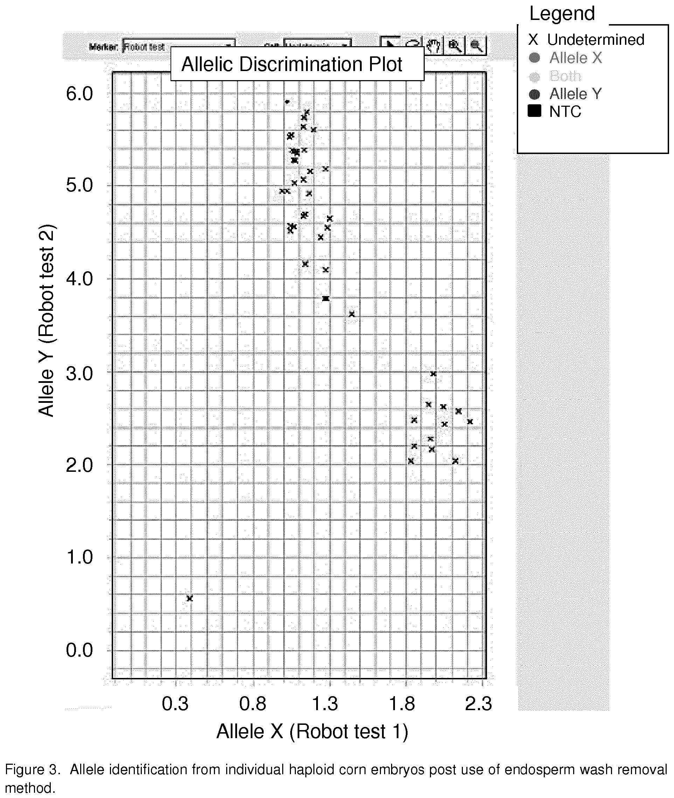

DNA extraction and marker analysis were performed as described under experiment 1, but the embryos were soaked in 10 mM NaOH. The result for the marker analysis is shown in Fig. 3.

### Endosperm Wash Method 2

This endosperm washing method 2 was performed as in endosperm wash method 1 (50ml tubes with heavy shaking), but endosperm tissue was removed by washing embryos in MS buffer plus 3% sucrose for 30 minutes.

DNA was extracted as described under endosperm wash method 1.

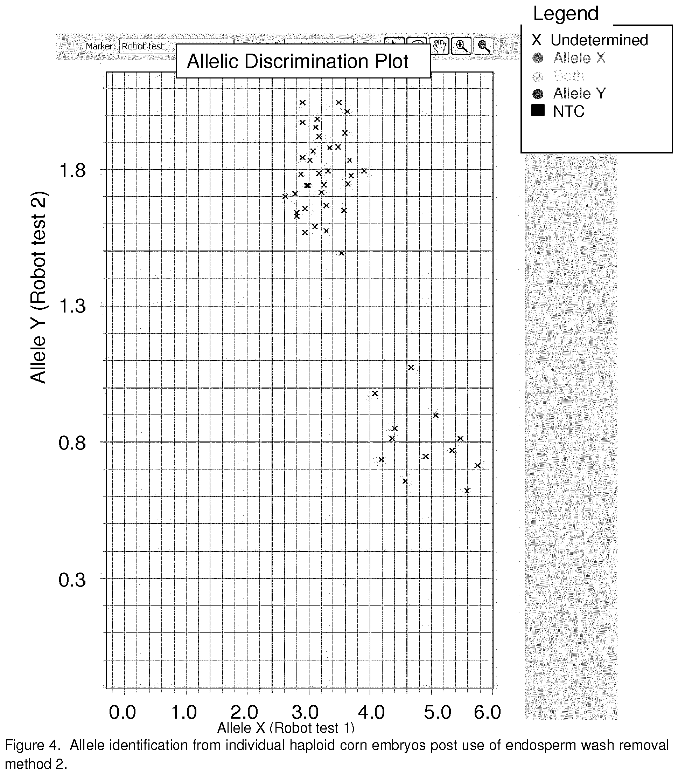

The result for the molecular marker analysis is shown in Fig. 4.

**Table 2. Embryo germination rates observed from embryos soaked in NaOH and using endosperm wash methods 1 & 2.**

| **Embryo Ploidy / Parent Source** | **Endosperm Wash Method** | **Initial Number of Embryos** | **Number Germinated Embryos** | **Percent Embryo Germination** |
|---|---|---|---|---|
| Haploid, Parent1 | Control | 42 | 36 | 86% |
| Haploid, Parent2 | Control | 26 | 25 | 96% |
| Haploid, Parent1 | Endosperm Wash Method 1 | 28 | 23 | 82% |
| Haploid, Genotype1 | Endosperm Wash Method 2 | 29 | 27 | 93% |
| Haploid, Parent2 | Endosperm Wash Method 1 | 38 | 38 | 100% |
| Haploid, Parent2 | Endosperm Wash Method 2 | 27 | 24 | 89% |

### Example 5. Soaking of Diploid Embryos in NaOH to Facilitate DNA Extraction.

Example 5 Removal of endosperm tissue was done as described in Example 4.

Each embryo was placed into a well of a 96 well block, to each embryo 200 µl of a 20 mM NaOH solution was added. The 96 well block was placed on a horizontal shaker, the blocks were slowly agitated for 1 hour. After 1 hour, 160 µl of the NaOH solution from each well were transferred into the wells of a second block and 1M TrisHCl (or any other pH adjusting chemical which does not harm downstream application of the DNA) added to lower the pH to around 8 for PCR. Other downstream applications may require different pH adjustments. The embryos were washed several times in sterile water and 0.5 x TE.

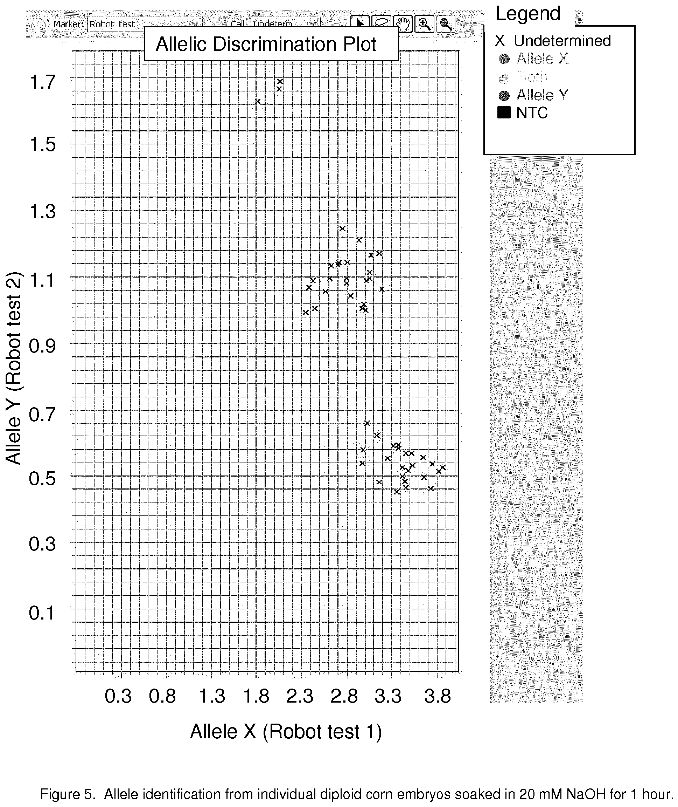

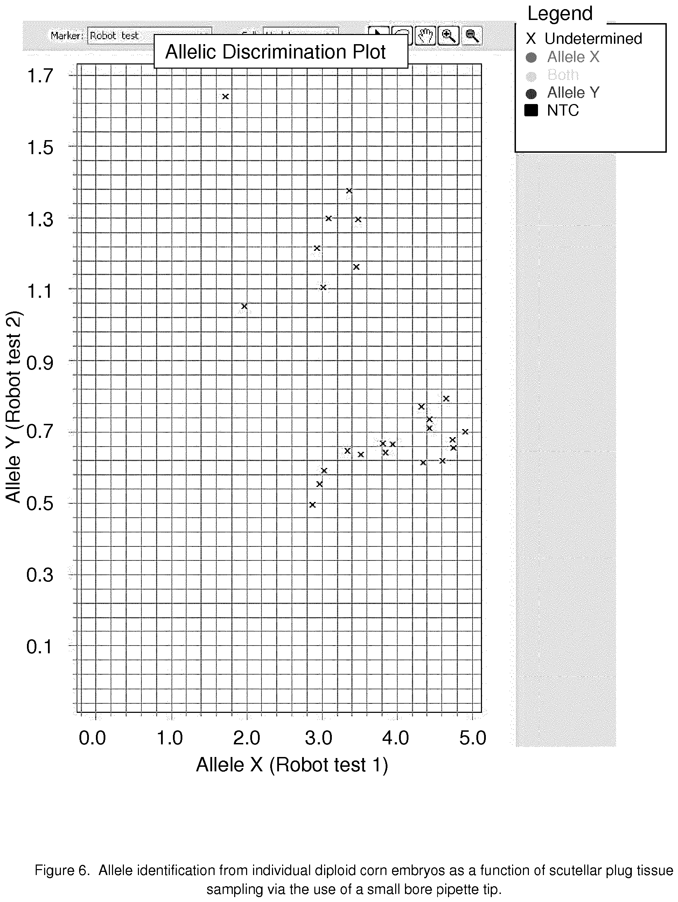

For molecular marker analysis, the DNA from 24 embryos which was released into the soaking solution was used directly as a template for Taqman PCR reaction. Results are shown in Fig.5

**Table 3. Embryo germination rates observed from diploid embryos soaked in 20 mM NaOH for one hour.**

| **Embryo Ploidy** | **DNA Extraction Method** | **Initial Number of Embryos** | **Number Germinated Embryos** | **Percent Embryo Germination** |
|---|---|---|---|---|
| Diploid | Control | 50 | 48 | 96% |
| Diploid | DNA Extracted by Soaking in NaOH | 48 | 46 | 96% |

### Example 6. Removal of Immature Corn Scutellar Tissue from Haploid and Diploid Immature Corn Embryos With A Small Bore Pipette Tip.

### Experiment 6

Scutellar tissue from immature diploid and haploid corn embryos (approximately 3 to 6 mm) was sampled by penetrating the scutellum with a 2 µl pipette tip (VWR North America Cat. No. 53509-130). Scutellum tissue containing pipette tips were placed vertically into wells of 96 well blocks. To facilitate the removal of the tissue plug, 50 µl of 30mM NaOH was added. The pipette tips remained in the NaOH solution for 1 hour. After 1 hour each tissue plug was released into the NaOH solution via positive air pressure. The plugs were kept one hour in NaOH to disrupt the cells further and release the DNA into the NaOH solution. Finally 1 M TrisHCl (or any other pH adjusting chemical which does not harm downstream application of the DNA) was added to lower the pH to around 8 for PCR. Other downstream applications may require different pH adjustments. Any other cell disrupting agent (e.g KOH) could be added or the cells of the plug could be disrupted mechanically e.g. with a steel bead or a steel pin. The DNA could be used directly as template for PCR reactions or the DNA or additional cleaning steps could be applied. Germination evaluations were conducted as previously described in experiments 1 and 2.

**Table 4. Embryo germination rates observed in diploid embryos post scutellar tissue sampling with a 2 µl pipette tip.**

| **Embryo Ploidy** | **DNA Extraction Method** | **Initial Number of Embryos** | **Number Germinated Embryos** | **Percent Embryo Germination** |
|---|---|---|---|---|
| Diploid | Control | 25 | 25 | 100% |
| Diploid | Scutellar Plug Sampling | 25 | 25 | 100% |

**Table 5. Embryo germination rates observed from haploid embryos post tissue sampling with a 2 ul pipette tip.**

| **Embryo Ploidy** | **DNA Extraction Method** | **Initial Number of Embryos** | **Number Germinated Embryos** | **Percent Embryo Germination** |
|---|---|---|---|---|
| Haploid | Control | 643 | 615 | 96% |
| Haploid | Scutellar Plug Sampling | 276 | 264 | 96% |

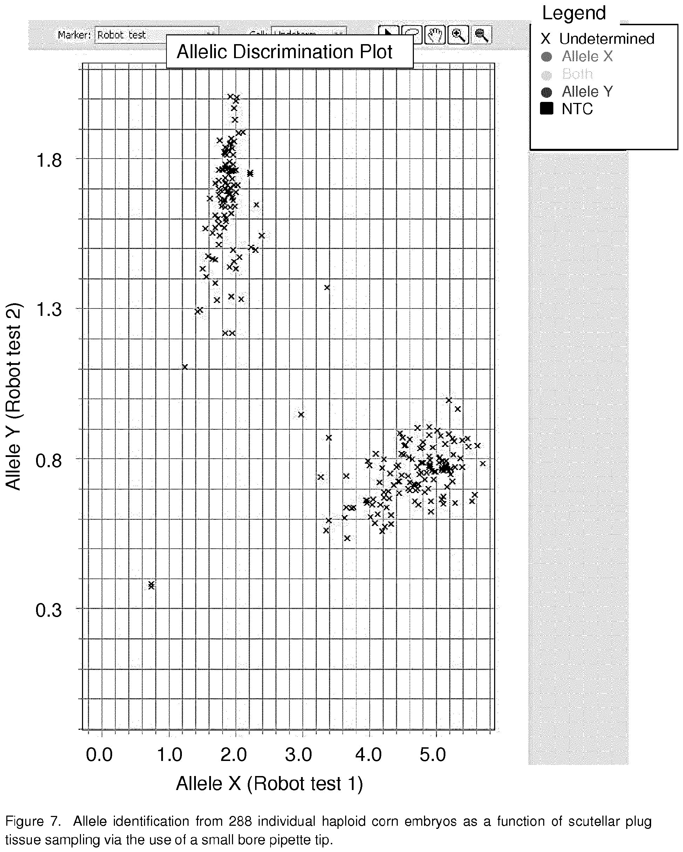

For molecular marker analysis, the DNA was used directly as a template for Taqman PCR reaction. Results for 288 haploid DNA samples are shown in Fig. 7. Results for diploid DNA samples are shown in Fig, 6.

### Example 7. Removal of Immature Corn Scutellar Tissue from Haploid Immature Corn Embryos By Scutellum Penetration With a Sharp Tip.

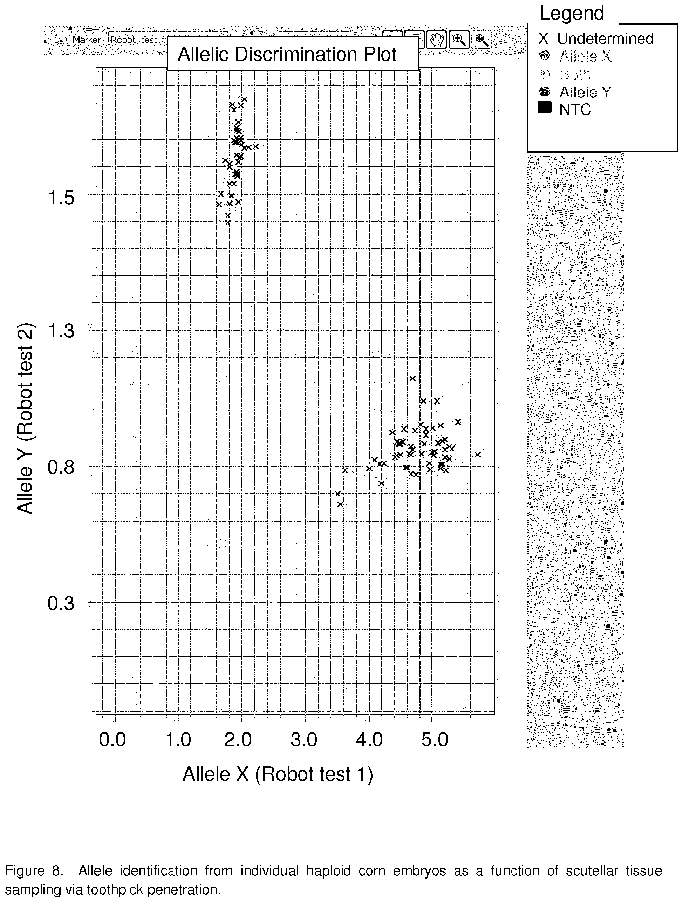

Experiment 8. Tissue/DNA extraction from haploid immature corn embryo scutellar tissue via penetration with a toothpick. Toothpick tips were sharpened to a narrow point and used to penetrate into and through the scutellar surface. The sharpened toothpick ends were then placed vertically into wells of 96 well blocks. 50 µl of 30 mM NaOH was added to each well. Any other solutions which do not interfere negatively with downstream applications of the isolated DNA could be used. The scuttelum tissue was released mechanically by rubbing the toothpick tips against the well walls. 1 M TrisHCl was added (or any other pH adjusting chemical which does not harm downstream application of the DNA) to lower the pH to around 8 for PCR. Other downstream applications may require different pH adjustments.. For molecular marker analysis, the DNA was used directly as a template for Taqman PCRs. Results for 96 DNA samples are shown in Fig. 8.

**Table 6. Embryo germination rates observed in haploid embryos post tissue sampling by toothpick scutellar penetration. Same control data was used in tables 5 and 6.**

| **Embryo Ploidy** | **DNA Extraction Method** | **Initial Number of Embryos** | **Number Germinated Embryos** | **Percent Embryo Germination** |
|---|---|---|---|---|
| Haploid | Control | 643 | 615 | 96% |
| Haploid | Toothpick Penetration | 103 | 97 | 94% |

### Example 8 Tissue/DNA extraction from Diploid Immature Corn Embryo Scutellar Tissue via penetration with a Toothpick.

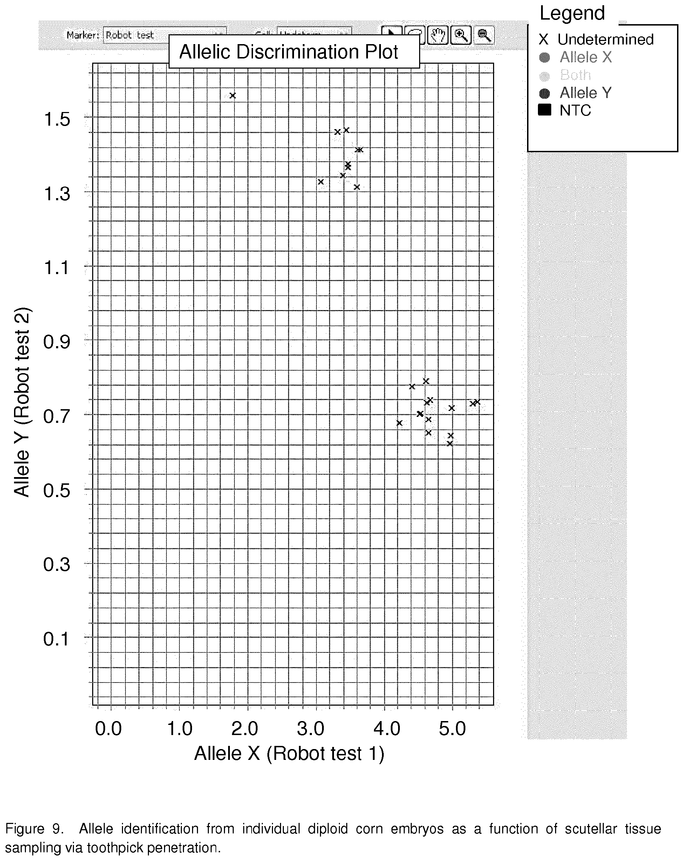

DNA Extraction: DNA extraction and molecular marker analysis from diploid embryos was done as described under experiment 8.Results for DNA samples are shown in Fig. 9

**Table 7. Embryo germination rates observed in diploid embryos post tissue sampling by toothpick scutellar penetration.**

| **Embryo Ploidy** | **DNA Extraction Method** | **Initial Number of Embryos** | **Number Germinated Embryos** | **Percent Embryo Germination** |
|---|---|---|---|---|
| Diploids | Control | 25 | 25 | 100% |
| Diploids | Toothpick Penetration | 25 | 25 | 100% |

### Example 9. Viability Evaluation of Germinated Embryos soaked in NaOH

Five days post germination, germinated embryos were washed in MS buffer plus 3% sucrose for 30 minutes as described in experiment 1. After 30 minutes the washing solution was poured off and 40 ml of 5 mM NaOH or 40 ml of 10 mM NaOH were added. The tubes were slowly agitated on a horizontal shaker. After 30 minutes, the embryos were removed, washed with MS + 3% and the germination capacity evaluated as described under example 2. The results show that late stage germinated embryos survive NaOH soaking.

**Table 8. Embryo germination rates observed from germinated haploid embryos post soaking in NaOH.**

| **Embryo Ploidy** | **DNA Extraction Method** | **Initial Number of Embryos** | **Number Germinated Embryos** | **Percent Embryo Germination** |
|---|---|---|---|---|
| Haploid | Germinated embryos soaked with 10mM NaOH | 13 | 11 | 85% |
| Haploid | Germinated embryos soaked with 5mM NaOH | 28 | 26 | 93% |

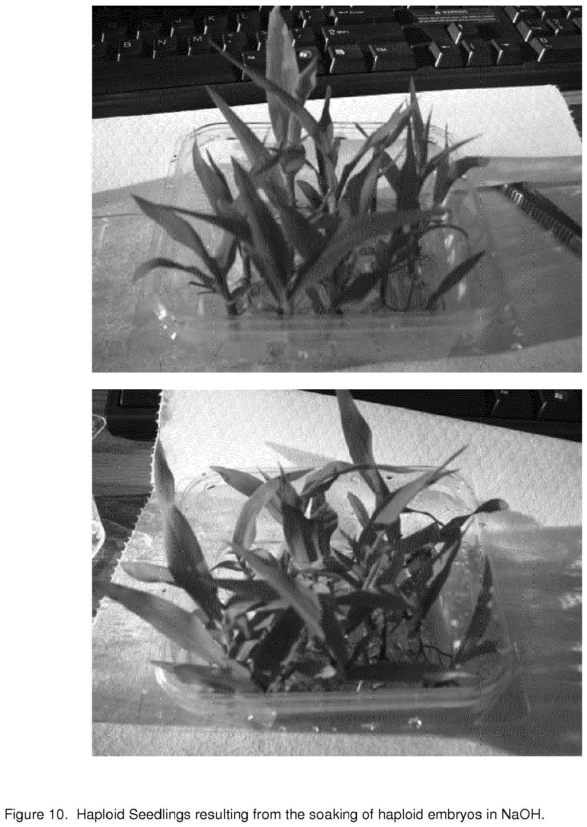

Pictures illustrating haploid seedlings derived the two NaOH treatments are found in Fig. 10.

### Example 10. Scutellar plug sampling and stasis storage.

Haploid embryos were identified 24 hours post extraction from corn kernels. Embryos were subsequently either 1) germinated on solidified MS medium or 2) tissue sampled via the collection of a tissue plug and germinated on solidified MS medium or 3) placed on an MS moistened filter paper in a petri dish which was then moved into a dark and cooled chamber (7C/45F) for a period of 8 days after which the embryos were germinated on solidified MS medium or 4) tissue sampled via the collection of a scutellar tissue plug and placed on an MS moistened filter paper in a petri dish which was moved into a dark cool (7C/45F) chamber for a period of 8 days after which the embryos were germinated on solidified MS medium. Surviving seedlings were transplanted into flats containing a Fafard potting mixture.

**Table 9. Seedling transplantation efficacy as a function of tissue sampling and stasis storage.**

| **Haploid Embryo Treatment** | **Initial No. of Embryos** | **No. of Transplanted Seedlings** | **Percent Seedlings Transplanted** |
|---|---|---|---|
| No Cold Storage | 50 | 35 | 70% |
| No Cold Storage and Scutellar Plug Sampling | 55 | 35 | 64% |
| Stasis Storage of Embryos | 58 | 37 | 64% |
| Scutellar Plug Sampling and Stasis Storage | 55 | 45 | 82% |

### Example 11. Soaking of Haploid Embryos in TrisHCl/SDS to Facilitate DNA Extraction.

Experiment 1. For the removal of adherent endosperm tissue, all embryos were pooled and transferred into a 50 ml tube containing 40 ml MS buffer and 3% sucrose. To enhance the washing efficiency, the tube was placed on a horizontal shaker and vigorously shaken for 40 minutes. After 40 minutes the washing solution was poured off and the embryos were briefly rinsed in H₂O.

For DNA extraction, each embryo was placed into a well of a 96 well block, to each embryo 60 µl of a solution consisting of 200 mM TrisHCl pH 9.2 and 0.0025 - 0.005 % SDS was added. The 96 well block was placed on a horizontal shaker, the blocks were slowly agitated for 1 hour. The TrisHCl/SDS solution disrupts scutellar or embryo axis cells, the DNA and other cellular content of the disrupted cells is released into the solution. The concentrations of the components in the soaking solution and the pH of the solution can be higher or lower and can easily be modified by the skilled person in the art. Also the soaking time can be shortened or prolonged. Any other chemical which disrupts cells but does not harm the viability of the embryos could be used or any chemical substances which do not harm the viability of the embryos could be added to a cell disrupting solution.

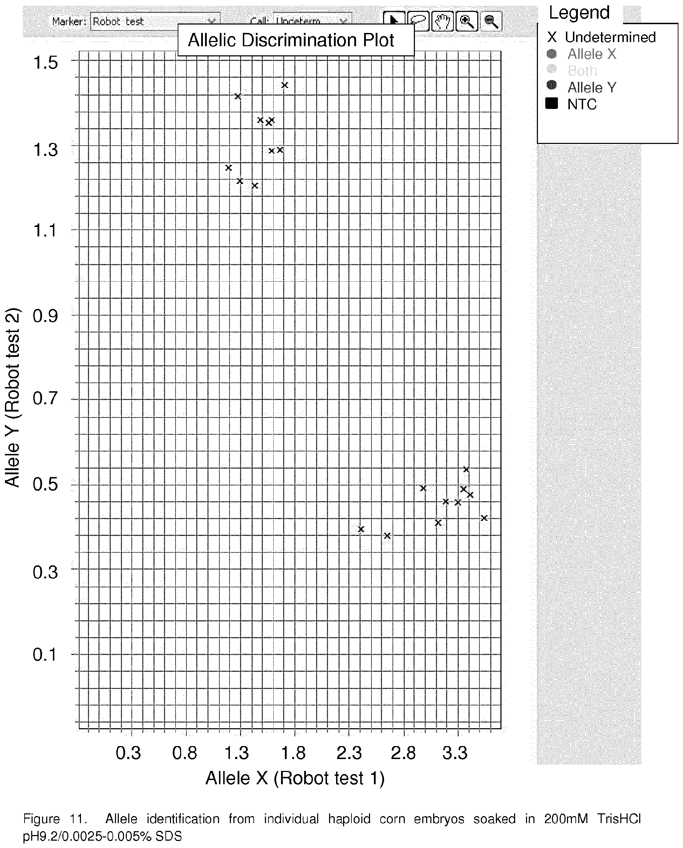

After 1 hour the soaking solution was transferred into a new 96 well block. The embryos were briefly washed in a solution of MS/3 % sucrose and transferred onto filter paper soaked in MS/3 % sucrose to compare germination rate to untreated embryos (= controls). Germination rate of control and TrisHCl/SDS treated embryos were comparable. For molecular marker analysis, the DNA from 20 embryos which was released into the soaking solution was used directly as a template for Taqman PCRs. Modification of the DNA (e.g. additional cleaning steps via alcohol precipitation) could be applied. Direct PCR results are shown in Fig.11

Experiment 2. For the removal of adherent endosperm tissue, all embryos were pooled and transferred into a 50 ml tube containing 40 ml MS buffer and 3% sucrose. To enhance the washing efficiency, the tube was placed on a horizontal shaker and vigorously shaken for 40 minutes. After 40 minutes the washing solution was poured off and the embryos were briefly rinsed in H₂O.

After the washing step, some part of the scutellum tissue was removed from each embryo with a razor blade. The removed tissue was discarded and each embryo was placed into a well of a 96 well block. To each embryo 60 µl of a solution consisting of 200 mM TrisHCl pH 9.2 and 0.005 % SDS was added. The 96 well block was placed on a horizontal shaker, the blocks were slowly agitated for 1 hour. The TrisHCl/SDS solution disrupts scutellar and/or embryo axis cells, the DNA and other cellular content of the disrupted cells is released into the solution. The concentrations of the components in the soaking solution and the pH of the solution can be higher or lower and can easily be modified by the skilled person in the art. Also the soaking time can be shortened or prolonged. Any other chemical which disrupts cells but does not harm the viability of the embryos could be used or any chemical substances which do not harm the viability of the embryos could be added to a cell disrupting solution.

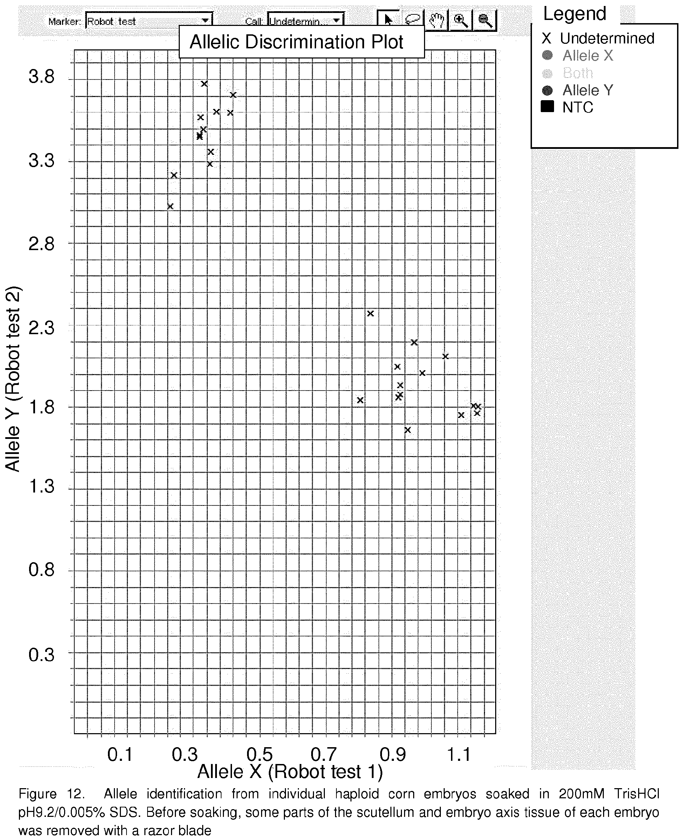

After 1 hour the soaking solution was transferred into a new 96 well block. The embryos were briefly washed in a solution of MS/3 % sucrose and transferred onto filter paper soaked in MS/3 % sucrose to compare germination rate of control untreated embryos. Germination rate of controls and treated were comparable. For molecular marker analysis, the DNA from 20 embryos which was released into the soaking solution was used directly as a template for Taqman PCRs. Modification of the DNA (e.g. additional cleaning steps via alcohol precipitation) could be applied. Direct PCR results are shown in Fig.12

### Example 12. Soaking of Haploid Embryos in Cellulase to Facilitate DNA Extraction.

Experiment 1. For the removal of adherent endosperm tissue, all embryos were pooled and transferred into a 50 ml tube containing 40 ml MS buffer and 3% sucrose. To enhance the washing efficiency, the tube was placed on a horizontal shaker and vigorously shaken for 40 minutes. After 40 minutes the washing solution was poured off and the embryos were briefly rinsed in H₂O.

For DNA extraction, each embryo was placed into a well of a 96 well block, to each embryo 60 µl of a solution consisting of 10mM of a Sodium Phosphate buffer pH 7.0 and > 1 - 5U of cellullase (Cellulase from Aspergillus sp., Sigma) was added. The 96 well block was placed on a horizontal shaker, the blocks were slowly agitated for 1 hour. Cellulase disrupts scutellar or embryo axis cells, resulting in the release of cellular content from the disrupted cells into the solution. The concentrations of the components in the soaking solution can be higher or lower and can easily be modified by the skilled person in the art. Also the soaking time can be shortened or prolonged. Any other chemical which does not harm the viability of the embryos and has no major negative effect on the activity of the enzyme could be added to the enzyme solution.

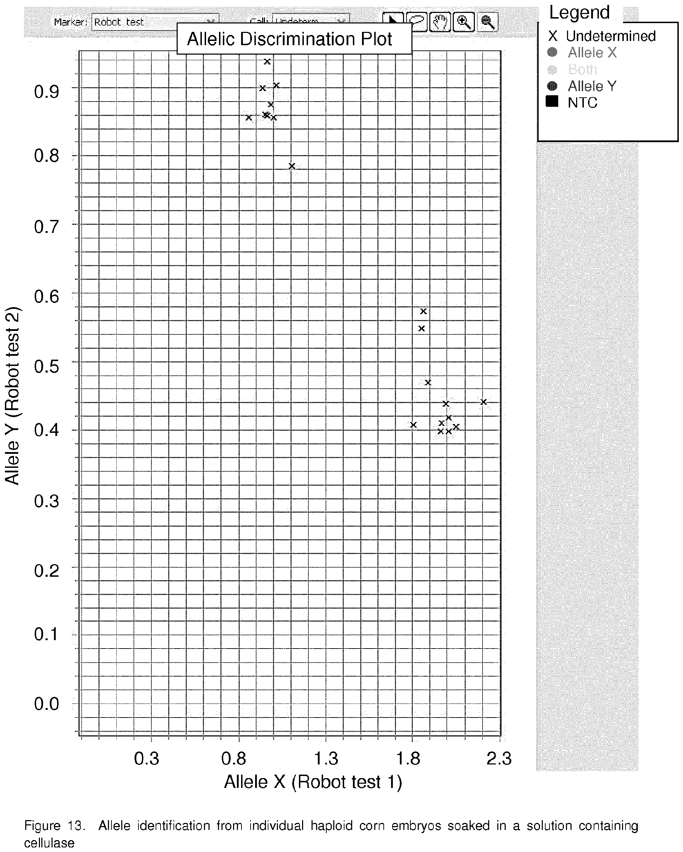

After 1 hour the soaking solution was transferred into a new 96 well block. The embryos were briefly washed in a solution of MS/3 % sucrose and transferred onto filter paper soaked in MS/3 % sucrose to compare germination rate to untreated embryos (= controls). Germination rate of controls and treated were comparable. For molecular marker analysis, the DNA from 20 embryos which was released into the soaking solution was used directly as a template for Taqman PCRs. At this point, any DNA cleaning processes could be applied. Modification of the DNA (e.g. additional cleaning steps via alcohol precipitation) could be applied. Direct PCR results are shown in Fig.13

### Example 13. Soaking of Diploid Embryos in Pectinase to Facilitate DNA Extraction.

Experiment 1. For the removal of adherent endosperm tissue, all embryos were pooled and transferred into a 50 ml tube containing 40 ml MS buffer and 3% sucrose. To enhance the washing efficiency, the tube was placed on a horizontal shaker and vigorously shaken for 40 minutes. After 40 minutes the washing solution was poured off and the embryos were briefly rinsed in H₂O.

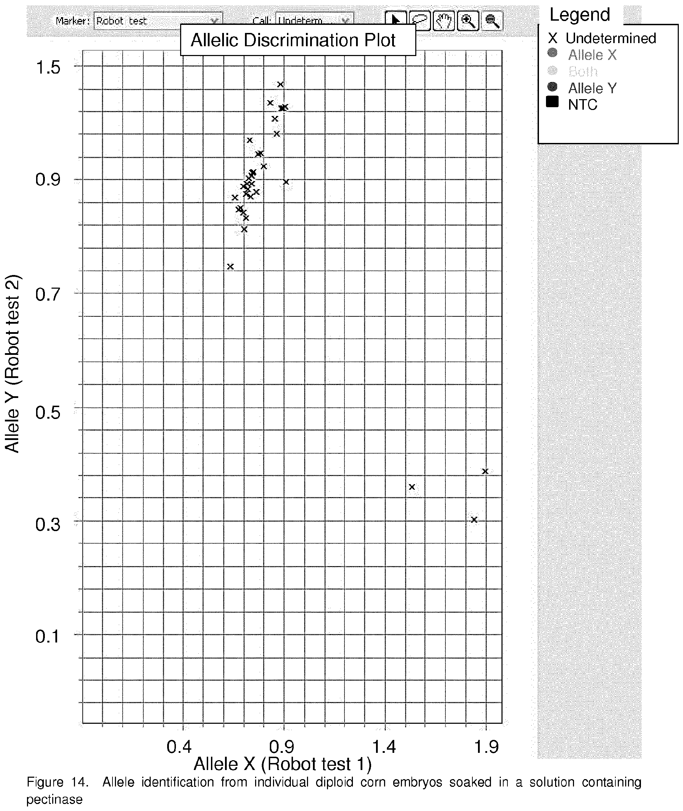

For DNA extraction, each embryo was placed into a well of a 96 well block, to each embryo 60 µl of a solution consisting of 10mM of a Sodium Phosphate buffer pH 7.0 and > 1 - 5U of pectinase (Pectinase from Aspergillus aculeatus, Sigma) was added. The 96 well block was placed on a horizontal shaker, the blocks were slowly agitated for 1 hour. Pectinase disrupts scutellar or embryo axis cells, resulting in the release of cellular content from the disrupted cells into the solution. The concentrations of the components in the soaking solution can be higher or lower and can easily be modified by the skilled person in the art. Also the soaking time can be shortened or prolonged. Any other chemical which does not harm the viability of the embryos and has no major negative effect on the activity of the enzyme could be added to the enzyme solution. After 1 hour the soaking solution was transferred into a new 96 well block. The embryos were briefly washed in a solution of MS/3 % sucrose and transferred onto filter paper soaked in MS/3 % sucrose to compare germination rate to untreated embryos (= controls). Germination rate of controls and treated were comparable. For molecular marker analysis, the DNA from 20 embryos which was released into the soaking solution was used directly as a template for Taqman PCRs. At this point, any DNA cleaning processes could be applied. Modification of the DNA (e.g. additional cleaning steps via alcohol precipitation) could be applied. Direct PCR results are shown in Fig.14.

## Claims

1. A non-destructive, viability retaining method of sampling an immature plant embryo, comprising the steps of: removing a tissue sample comprising at least a portion of the scutellum of the plant embryo;
wherein the obtained cellular content via tissue sampling is subjected to genetic, chemical or biochemical analysis, and
wherein the extracted embryos are put into stasis storage to achieve a quiescent condition until the outcome of the genetic, chemical or biochemical analysis is known.

2. The method of sampling a plant embryo according to the previous claim, wherein said method is automated or semi-automated.

3. The method according to claims 1 or 2, wherein the sample removed comprises tissue from the non-coleoptilar end of the embryo.

4. The method according to claims 1 or 2, wherein the sample removed comprises tissue from the equator or middle region of the embryo.

5. A method of data driven plant advancement, comprising the steps of
a. Inducing haploids;
b. Extracting and identifying immature plant embryos from amongst a mixed population of diploid and haploid embryos;
c. sampling the immature plant embryos according to the method of claims 1-4; and
d.Optionally, the application of a doubling chromosome process.

6. A method of data driven plant advancement according to claim 5, wherein the embryos are stored in a viability retaining fashion in a controlled atmosphere temperature and nutritional environment.

7. A method of data driven plant enhancement according to any of claims 5 to 6, wherein
a. The embryo is manipulated using a robotic arm;
b. A camera is used to select haploids or non-haploids; and
c. A camera or vision system is used to guide the sampling instrument to the tissue to be sampled.

## Patentansprüche

1. Zerstörungsfreies, Lebensfähigkeit erhaltendes Probenahmeverfahren für unreife Pflanzenembryonen, umfassend die folgenden Schritte: Entnehmen einer Gewebeprobe umfassend wenigstens einen Teil des Scutellums des Pflanzenembryos;
wobei der erhaltene zelluläre Inhalt über Gewebeprobenahme einer genetischen, chemischen oder biochemischen Analyse unterzogen wird, und
wobei die extrahierten Embryos in Staselagerung gegeben werden, um einen Ruhezustand zu erreichen, bis das Ergebnis der genetischen, chemischen oder biochemischen Analyse bekannt ist.

2. Probenahmeverfahren für Pflanzenembryonen nach dem vorhergehenden Anspruch, wobei das Verfahren automatisiert oder teilautomatisiert ist.

3. Verfahren nach Anspruch 1 oder 2, wobei die entnommene Probe Gewebe des nicht-koleoptilen Endes des Embryos umfasst.

4. Verfahren nach Anspruch 1 oder 2, wobei die entnommene Probe Gewebe der Äquator- oder mittleren Region des Embryos umfasst.

5. Verfahren zur datengesteuerten Pflanzenverbesserung, umfassend die folgenden Schritte:
a. Induzieren von Haploiden;
b. Extrahieren und Identifizieren unreifer Pflanzenembryonen aus einer gemischten Population diploider und haploider Embryonen;
c. Probenahme der unreifen Pflanzenembryonen nach dem Verfahren nach Anspruch 1-4; und
d. gegebenenfalls, Anwendung eines Chromosomenverdopplungsverfahrens.

6. Verfahren zur datengesteuerten Pflanzenverbesserung nach Anspruch 5, wobei die Embryonen in einer Lebensfähigkeit erhaltenden Weise in einer gesteuerten Atmosphärentemperatur und Ernährungsumgebung gelagert werden.

7. Verfahren zur datengesteuerten Pflanzenverbesserung nach Anspruch 5 und 6, wobei
a. der Embryo mithilfe eines Roboterarms gehandhabt wird;
b. eine Kamera verwendet wird, um Haploide oder Nicht-Haploide auszuwählen; und
c. eine Kamera verwendet wird, um das Probenahmeinstrument zu dem zu entnehmenden Gewebe zu leiten.

## Revendications

1. Procédé conservant la viabilité, non destructif, d'échantillonnage d'un embryon de plante immature, comprenant les étapes de : prélèvement d'un échantillon de tissu comprenant au moins une partie du scutellum de l'embryon de plante ;
dans lequel le contenu cellulaire obtenu par échantillonnage de tissu est soumis à une analyse génétique, chimique ou biochimique, et
dans lequel les embryons extraits sont placés en conservation en stase pour obtenir un état quiescent jusqu'à ce que le résultat de l'analyse génétique, chimique ou biochimique soit connu.

2. Procédé d'échantillonnage d'un embryon de plante selon la revendication précédente, ledit procédé étant automatisé ou semi-automatisé.

3. Procédé selon revendications 1 ou 2, dans lequel l'échantillon prélevé comprend du tissu de l'extrémité non coléoptilaire de l'embryon.

4. Procédé selon revendications 1 ou 2, dans lequel l'échantillon prélevé comprend du tissu de la région équatoriale ou centrale de l'embryon.

5. Procédé d'amélioration de plante guidée par données, comprenant les étapes de
a. induction d'haploïdes ;
b. extraction et identification d'embryons de plante immature parmi une population mixte d'embryons diploïdes et haploïdes ;
c. échantillonnage des embryons de plante immature selon le procédé des revendications 1 à 4 ; et
d. facultativement, application d'un processus de doublement de chromosomes.

6. Procédé d'amélioration de plante guidée par données selon la revendication 5, dans lequel les embryons sont stockés de façon à conserver la viabilité à une température d'atmosphère contrôlée et dans un environnement nutritif.

7. Procédé d'amélioration de plante guidée par données selon l'une quelconque des revendications 5 à 6, dans lequel
a. l'embryon est manipulé au moyen d'un bras robotique ;
b. une caméra est utilisée pour sélectionner des haploïdes ou non haploïdes ; et
c. une caméra ou un système de vision est utilisé pour guider l'instrument d'échantillonnage vers le tissu à échantillonner.
